(19) Europäisches Patentamt
European Patent Office
Office européen des brevets

(11) **EP 1 159 476 B1**

(12) **EUROPEAN PATENT SPECIFICATION**

(45) Date of publication and mention
of the grant of the patent:
**14.07.2004 Bulletin 2004/29**

(51) Int Cl.⁷: **D04H 1/48**, A44B 18/00

(86) International application number:
**PCT/SE1999/002130**

(21) Application number: **99962628.6**

(22) Date of filing: **19.11.1999**

(87) International publication number:
**WO 2000/031330 (02.06.2000 Gazette 2000/22)**

(54) **FASTENER MEANS**

SCHLIESSELEMENT

DISPOSITIFS DE FIXATION

(84) Designated Contracting States:
**AT BE CH CY DE DK ES FI FR GB GR IE IT LI LU MC NL PT SE**

(30) Priority: **19.11.1998 SE 9803970**

(43) Date of publication of application:
**05.12.2001 Bulletin 2001/49**

(73) Proprietor: **SCA Hygiene Products AB**
**405 03 Göteborg (SE)**

(72) Inventor: **MAGNUSSON, IngBritt**
**S-435 37 Mölnlycke (SE)**

(74) Representative: **Larsson, Karin et al**
**Albihns Stockholm AB,**
**Box 5581**
**114 85 Stockholm (SE)**

(56) References cited:
**EP-A1- 0 765 616          EP-A2- 0 258 015**
**EP-A2- 0 780 505          WO-A1-95/07677**
**WO-A1-95/17111**

• **DATABASE WPI Week 1994, Derwent Publications Ltd., London, GB; AN 1994-080418, XP002949692 & JP 6 033 359 A (KURARAY CO LTD) 08 February 1994 & PATENT ABSTRACTS OF JAPAN & JP 06 033 359 A (KURARAY CO LTD) 08 February 1994**

**Description**

The present invention relates to a material that can function as a fastener surface for the male component of a hook and loop fastener system, to a method of producing said material, and to an absorbent article, such as a diaper, in which the material is used as fastener means, and to a use of said material as female component in a hook and loop fastener system in an absorbent article.

In absorbent articles, such as diapers and incontinence guards for instance, the article is often secured to the wearer with the aid of a hook and loop fastener system. Such fastener means comprise a male and a female component, where the former is comprised of hooks and the latter is comprised of loops. These two fastener components can be fastened together and thereafter separated from one another.

The fastener devices comprise loops and hooks formed on one side of a fibre tape which is bonded to a carrier tape. The opposite surface of the carrier tape is suitably adhesive, so as to enable the tape to be fastened easily to an article.

The described two-layer construction that includes a carrier is necessary in order to ensure that the fastener device will be sufficiently strong and can be fastened to an absorbent body, e.g. glued thereto.

Known loop-carrying tape material is formed, e.g., by gluing a loop-carrying fibrous web to a carrier web, by needling loops from a fibrous web through a carrier web, or by melting one surface of a loop-carrying fibrous web that includes hot hotmelt fibres , so as to obtain a coherent carrier surface. Such materials are described in, for instance, EP-A2-0780505, forming the preamble of claim 1, or WO96/04812, WO95/33390, WO92/20251, US-A-3,694,867, EP-A2-0258015, WO95/17111 and EP-A1-0765616.

One drawback with such fastener devices is that the laminate construction is not cost-effective, due to material consumption on the one hand and to the fact that several process steps are required on the other hand. Furthermore, the fastener device can be unnecessarily rigid and therefore uncomfortable, and may even chafe the wearer's skin. However, fastener devices of this type known hitherto have always required a carrier in order to be used and applied to an absorbent article, such as a diaper or an incontinence guard, and to bind together sufficiently strongly to ensure that the loops will not separate from the material as a hook and loop fastener is fastened to the material and then released and refastened at one and the same place or at some other place.

The object of the present invention is to provide a fastener device which is less expensive and more pliable than earlier known devices and the material of which is sufficiently stable for use as a female component to the male component of a hook and loop system.

This object is achieved in accordance with the invention with a fastener device that consists of a nonwoven fabric comprised of a carded fibrous web of functional polymer fibres and binding fibres that are bound together mechanically by needling. Additional binding is effected by partially melting the binder fibres. This results in a relatively dense material that has a sufficiently open structure to enable the male component of a hook and loop system to fasten therein. The fastener device is also sufficiently stable to prevent the fibres from being torn away from the surface as the hook component of the fastener is released. It is also sufficiently stable for the hook component of the fastener to be fastened, released from the fastener device and moved to a new position thereon.

The nonwoven fabric can thus be used instead of the conventional looped female component of a hook and loop fastener system. The nonwoven fabric, however, has no loops. On the other hand, the fastener surface is slightly rugged.

The carded fibre mixture will conveniently comprise 10-25% binding fibres and 75-90% functional fibres. The functional fibres will preferably consist of a mixture of two or more types of fibre, suitably 40-60% of one type and 60-40% of another type. The functional fibres are either crimped single-component fibres or spiralled bi-component fibres or multi-component fibres of the side-by-side type. There may be used a mixture of several types of crimped fibres, crimped and spiralled fibres, or several types of spiralled fibres. The spiralled fibres may have already be spiralled when preparing the fibre mixture to be carded and needled. It is also possible to use multi-component fibres of the side-by-side type that are spiralled during manufacture of the fastener device, conveniently when applying heat so as to partially melt the binding fibres.

It is also possible to use different polymer materials in the fibres of the inventive fastener device. Polyester fibres and polypropylene fibres are preferred. In bi-component fibres or multi-component fibres there can be used two types of polyester that have mutually different melting and expansion coefficients, or, e.g., polyester as onc component and another polymer material, e.g. polypropylene, as other components. The binding fibres may comprise bi-component fibres of polypropylene and polyethylene.

Different types of functional polyester fibres having varying lengths, thicknesses, etc., may be used in the manufacture of the inventive fastener device. A suitable thickness of the functional fibres is 1-6 dn (1,1-6,6 dtex), particularly 1.5-6 dn (1,65-6,6 dtex). When using only one type of fibre, such as polyester type fibres, fibres of two different thicknesses may be mixed together. A suitable length of both binding fibres and functional fibres is 30-80 mm, preferably 40-70 mm and then particularly about 60 mm.

Spiralled fibres may also be included in the mixture, to create a surface structure.

The polyester fibre fabric according to the invention can be used directly as fastener means and glued directly to a diaper for instance, in the absence of an intermediate carrier.

According to one preferred embodiment of the invention, the polyester nonwoven material includes spiralled fibres. The spiralled fibres that can be used in the inventive fastener device are suitably of the same type as those described in SE 9604833-5. These fibres are comprised of heat-crimped, spiralled, elastic thermoplastic multi-component fibres, preferably bi-component fibres. The components in the fibres are suitably disposed side-by-side. As the fibres are heat-treated the various components shrink to mutually different extents and thereby form the spiralled fibre.

In accordance with the invention, binding of the material can be further improved by calendering the material, i.e. with the aid of pressure and heat. Smooth-calendering is used in particular on one side of the material.

The inventive nonwoven fabric will now be described with reference to particular embodiments thereof and also with reference to the accompanying drawings, in which

Figs. 1-7 are schematic illustrations of the various steps that are carried out when testing the adhesiveness or holding strength of a hook and loop system;

Fig. 8 is a schematic view from above of a diaper in which the inventive nonwoven fabric is used as a tape landing zone system; and

Figs. 9 and 10 show parts of a diaper or incontinence guard that include a replaceable absorbent part.

Manufacture of the inventive fastener device is commenced by forming a nonwoven fabric from binding fibres and functional fibres. These fibres are then bound mechanically, suitably by needling, until there is obtained a relatively dense material that has a structure which is sufficiently open for the male component of a hook and loop fastener system to fasten therein and which is sufficiently stable to prevent an excessive number of fibres being loosened from the fastener device as the male component of said device is pulled away. Needling imparts to the material a slightly rugged surface that functions as a fastening surface. The material is then generally equilateral. The material is then heated so as to partially melt the binding fibres and to hold the fastener device together. Any multi-component fibres of the side-by-side type present will be spiralled in this heating process. As the fibres spiral, the needled nonwoven fabric will shrink to some extent and the ruggedness of the surface increase. One side of the material is then suitably smooth calendered. The smooth surface of the material thus obtained facilitates gluing of said surface to an article. This smooth calendering of said surface shall not be confused with the heat smelting process described in, e.g., EP-A1-0 780 505 for forming a carrier surface.

It is necessary to establish the extent to which needling shall be carried out, by experimenting with the material produced. In addition to a subjective assessment of the density of the material, its adhesiveness, and integrity, the shear force and peeling force required to release the fastener device from a hook-carrying part of the device can be measured in accordance with the following.

In the following description of the tests carried out, the female and male components of the hook and loop fastener systems are referred to as loop components and hook components respectively, regardless of whether these fastener parts present respectively loops and hooks as in a typical loop and hook fastener system or whether they lack the presence of loops, for instance, such as in the case of the inventive nonwoven fabric that includes a fastener surface.

**DESCRIPTION OF EMBODIMENTS**

Tests were carried out with the following mixtures.

| PARAMETERS | Test 1, 08-370 | Test 2, 08-378 | Test 3, 08-379 |
|---|---|---|---|
| Binding fibers % | 15 % 4 dn (4,4 dtex) bico | 20 % 2 dn (2,2 dtex) (2,2 dtex) bico | 20 % 2 dn (2,2 dtex) bico |
| Supplier | UNITIKA | HOECHST | HOECHST |
| Type | Melty 4080 | Trevira T 254 | Trevira T 254 |
| Functional fibres | 50 % 3 dn (3,3 dtex) | 40 % 3 dn (3,3 dtex) | 40 % 6dn (6,7 dtex) conj.hollow |
| Supplier | HOECHST | HOECHST | NAN YA |
| Type | Trevira T 290 | Trevira T 290 | |
| Functional fibres | 35 % 1,5 dn (1,7 dtex) | 40 % 3 dn (3,3 dtex) spiral | 40 % 3 dn (3,3 dtex) spiral |
| Supplier | HOECHST | RHONE-POULENC | RHONE-POULENC |

(continued)

| PARAMETERS | Test 1, 08-370 | Test 2, 08-378 | Test 3, 08-379 |
|---|---|---|---|
| Type | Trevira T 290 | Tergal X443 | Tergal X403 |

Tergal X443 and Tergal 403 are bi-component fibres of the side-by-side type that spiral when heated. The two components are comprised of two types of polyester that have mutually different melting points. NAN YA is a fibre that has already been spiralled and which also comprises bi-component polyester of the side-by-side type. All of the binding fibres are of the kind that includes a polyester core and a co-polyester casing.

As will be seen, two crimped polyester fibres of mutually different thickness were used in Test 1. In Test 2, there was used a crimped polyester fibre in mixture with a bi-component fibre that spiralled when heated. In Test 3, there was used a polyester fibre that had already spiralled in mixture with a bi-component fibre that spiralled when heated. A bi-component fibre of the type that includes a core which melts at high temperature and a casing which melts at low temperature were used as binding fibre in all tests. The material was heated until the binding fibre casing melted. but was stopped before the core melted.

| Samples | Description |
|---|---|
| EKL, loop material | Standard knitted polyester fabric coated with polypropylene, weight per unit area 90g/m$^2$ (PET 47.5, PP 42.5 g/m$^2$) |
| 08-370 K | Smooth calendered, long hotmelt fibres |
| 08-378, thick fibres | Side-by-side fibres that shrink and spiral in-line |
| 08-379, fine fibres | Side-by-side fibres that shrink and spiral in-line |

**DETERMINING THE SHEAR FORCE OF A HOOK AND LOOP FASTENER SYSTEM**

**Principle**

The hook material and the loop material are joined together in a controlled fashion.
The shear force is then measured with a tensile testing device.

**Sample preparation**

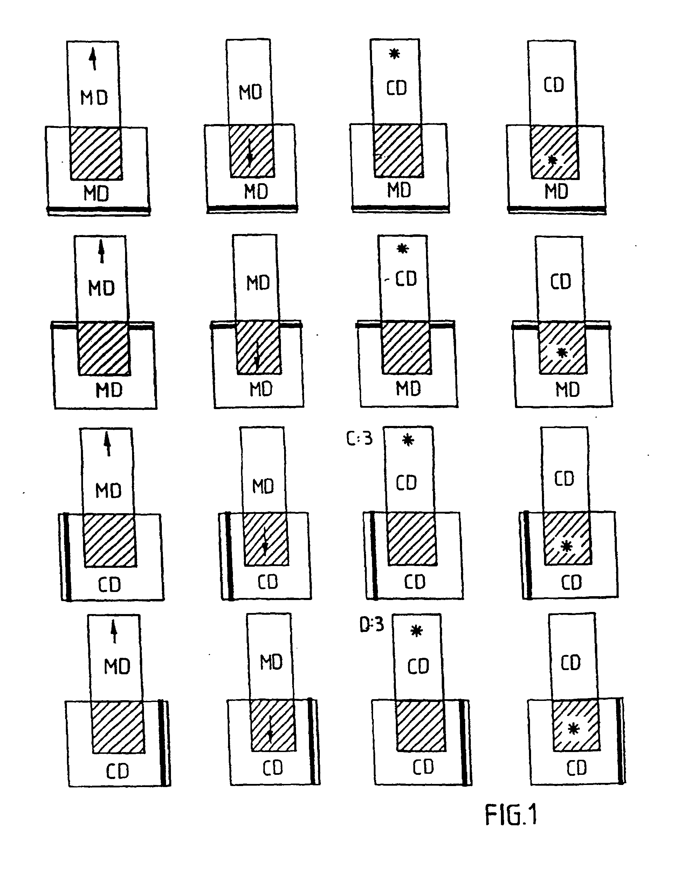

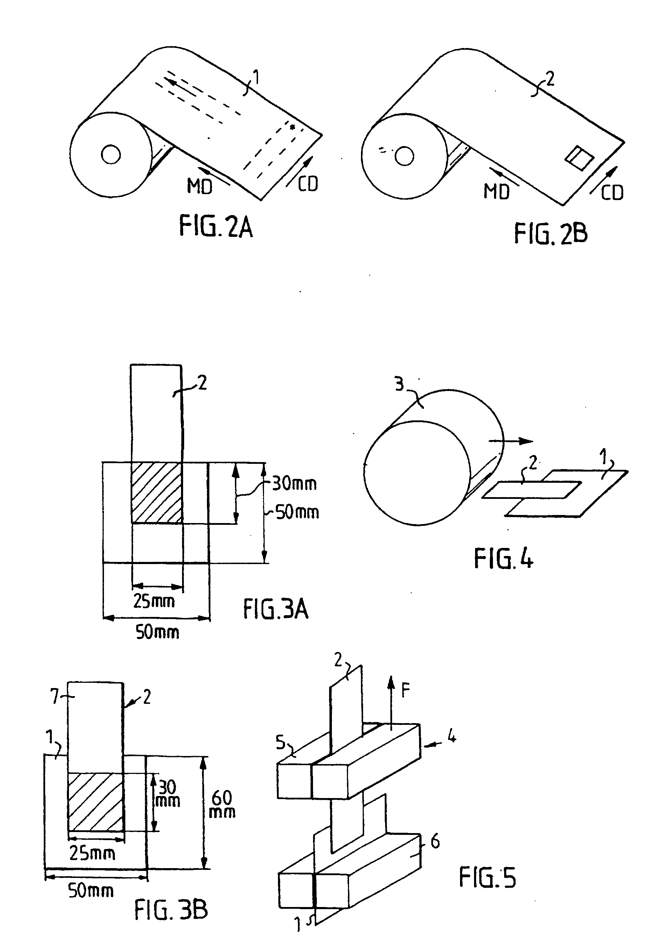

- Choose sample combination according to Fig. 1. MD denotes machine direction and CD denotes cross direction.
- Punch out the loop samples, 50 x 60 mm and mark with a pen according to Figs. 2A, B. Fig. 2A shows a roll of loop material 1 and Fig. 2B shows a role of hook material 2.
- Cut out the hook samples, 25 x 80 mm, and mark with an asterisk and arrow respectively, to show respective directions on the sample.
- Make a mark 30 mm in from the edge of the hook sample in accordance with Fig. 3A. The CD-directions shall be tested primarily.

**Procedure**

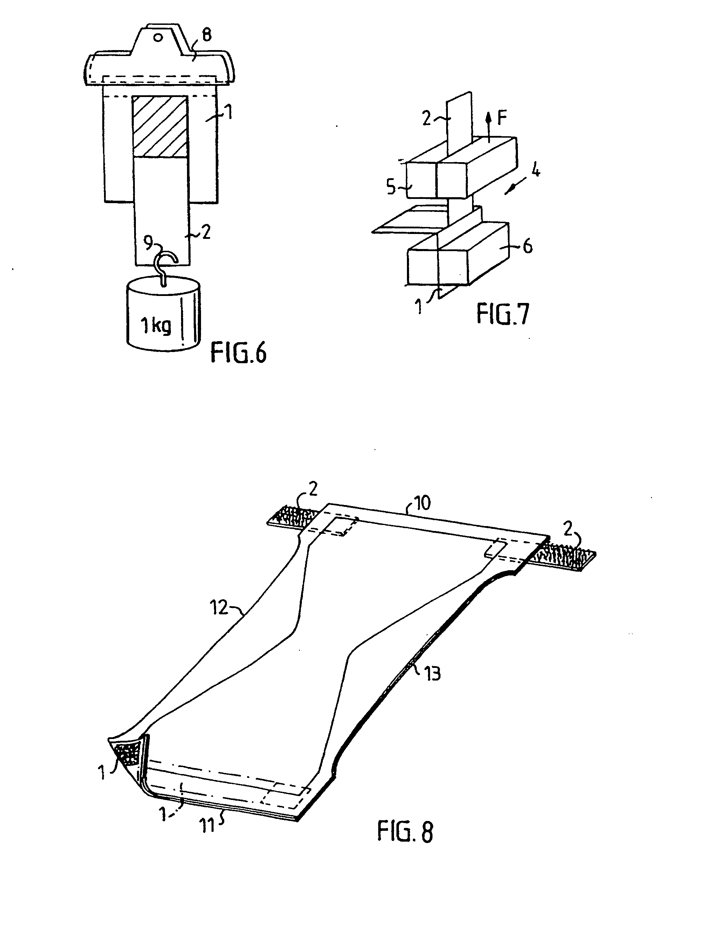

- Place the hook sample 2 carefully over the loop sample 1. The contact surface shall measure 30 x 25 mm. See Fig. 3A.
  Place the hook and loop sample 2 and 1 respectively in the roll apparatus and allow the pressure roll 3 to roll forwards and backwards one time (one cycle); see Fig. 4.
- Place the whole of the sample in the tensile testing device 4 with the hook material 2 in the upper clamp 5 and the loop material 1 in the lower clamp 6, as shown in Fig. 5. The materials are pulled in the direction of the arrow F.
- Continue with the test until the materials are fully "delaminated".

**Calculations and results**

$T_{max}$ =     Shear force, $N/cm^2$
$F_{max}$ =     The highest force detected during "delamination", N
l =     The length of the contact surface, mm
b =     The width of the contact surface, mm

$$T_{max} = \frac{F\ max \times 100}{1 \times b}$$

**DETERMINING THE DELAMINATING FORCE OF A HOOK AND LOOP FASTENER SYSTEM**

**Principle**

The hook material and the loop material are joined together in a controlled fashion. The delaminating force is then determined with the aid of a tensile testing device with the material at 90°.

**Sample preparation**

- Select a sample combination according to Fig. 1.
- Punch out the loop samples 1, 50 x 60 mm, and mark with a pen according to Fig. 2A.
- Clip/cut out the hook samples 2, 25 x 80 mm, and mark with a star and an arrow respectively, so as to show respective directions on the sample according to Fig. 2B.
- Make a mark 30 mm from one edge of the hook sample 2 - see Fig. 3B.
  The CD directions shall be tested primarily.

**Procedure**

- Place over the hook sample 2 a piece of tape 7 which is sufficiently large to leave free a surface measuring 25 x 30 mm.
- Place the hook sample 2 over the loop sample 1. Leave 10 mm of the loop strip 3 for fastening the strip in the clamp; see Fig. 3B.
- Place the hook sample 2 + the loop sample 1 in the roll apparatus and allow the pressure roll 3 to roll forwards and backwards one time (one cycle) - see Fig. 4.
  Begin to roll in the direction shown in the Figure.
- In order to generate a defined shear force, place the outwardly projecting part of the loop material 1 in a clamp 8 and the outwardly projecting part of the hook material in a clamp 9, with a weight of 1 kg.
- Allow the weight to hang freely for 10 seconds; see Fig. 6.
- Place the sample in the tensile testing device 4 with the hook component 2 in the upper clamp 5 and the loop component in the lower clamp 6.
- Carry out the test on the two materials at an opening angle of 180°; see Fig. 7.
- The delaminating force can also be determined with repeated opening and closing of the two components if so desired. This can only be done if no deformation occurs in the material during the test.

**Calculation and results**

The method measures the highest peaks (max. 20) during the test.

$F_{max}$ N/25 mm =     The highest peak during the test
$F_{med}$ N/25 mm =     The mean value of all peaks during the test

In the described tests, the shear force with respect to the loop material will preferably lie between 40 and 100 $N/7.5\ cm^2$ and the delaminating force between 2 and 5 N/25 mm (mean load) and between 3 and 8 N/25 mm (peak load) respectively. Excessively high values mean that it is difficult to loosen the hook and loop components from one another without tearing said components or without removing a component from the underlying backing sheet, whilst excessively low values indicate insufficient fastening ability or holding strength. It will be seen from the following that the inventive material has the properties desired and surpasses the reference material EKL in several respects.

The results are shown in Table 2 below.

## Table 2

| Fastener Device | Weight per unit area | Shear force, N/7.5 cm² Peak load, C:3 | | | Delaminating force, N/25 mm Mean load, C:3 | | | Delaminating force, N/25 mm Peak load, C:3 | | |
|---|---|---|---|---|---|---|---|---|---|---|
| | g/m² | Mean | Max | Min | Mean | Max | Min | Mean | Max | Min |
| EKL | 90 | 70 | | | 3.5 | | | 5.5 | | |
| 08-370K | 60 | 82 | 89 | 75 | 3 | 4 | 3 | 5 | 6 | 4 |
| | 80 | 92 | 101 | 80 | 3.5 | 4 | 3 | 5.5 | 6.5 | 4.5 |
| | 100 | 47 | 57 | 21 | 2.5 | 3 | 2 | 4 | 6.5 | 3 |
| 08-378 | 80 | 73 | 77 | 68 | 4 | 5 | 3 | 6 | 7 | 5 |
| 08-379 | 80 | 43 | 48 | 40 | 2 | 2 | 1.2 | 3 | 4 | 2 |

EP 1 159 476 B1

The best shear force result was obtained with sample 08-370K, followed by 08-378, although this latter gave a somewhat higher delaminating force.

All tests were carried out with standard hook C200 from 3M.

Fig. 8 is a schematic illustration of a diaper with which the inventive fastener material can be used. The diaper includes an absorbent sheet which is disposed between a liquid-permeable inner sheet that lies proximal to a wearer in use and a liquid impermeable outer sheet, or backing sheet, which lies distal from the wearer in use. The diaper has a generally rectangular shape and is delimited by two short sides 10, 11 and two long sides 12, 13. Two fastener tapes that include hook material 2 are fastened to the outside of the diaper, at one short end 10, such that a free part of each fastener tape forms an extension of said short side, wherewith the hook material faces in the same direction as the inside surface of the diaper. Loop material 1 according to the invention is fastened to the outside of the diaper at the other short end 11, in each comer thereof. When placing the diaper on a wearer, the centre part of the diaper is curved around the wearer's crotch, so that the short sides 10, 11 will lie around the wearer's waist. The diaper is fastened by applying the tapes that include hook material 2 to the tapes that include loop material 1. By way of alternative, a strip of loop material can be placed along the full length of the short side, instead of using two pieces of loop material along the length of the short side 11. This is shown by a broken line in Fig. 8.

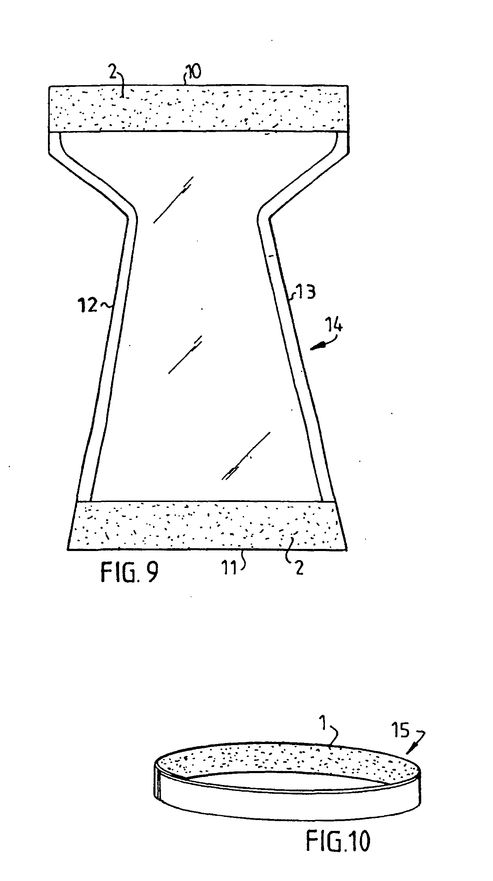

The inventive nonwoven fabric may also be used in the type of diaper or incontinence guard described in US-A-5,549,593, for instance. This type of diaper is illustrated in Figs. 9 and 10 and includes an absorbent part 14, the actual diaper, which is replaceable and secured to a belt 15 around the wearer's waist. The absorbent part 14 includes an absorbent layer disposed between a liquid-permeable inner sheet, or top sheet, which lies proximal to the wearer in use, and a liquid-impermeable outer sheet, or backing sheet, which lies distal from the wearer in use. Two strips of hook material 2 are disposed along each short side 10, 11 of the absorbent part 14, on the outer side thereof. A nonwoven fabric 1 that includes an inventive fastener surface in the form of loop material is provided on the inside of the belt 15. The short ends 10, 11 of the absorbent part 14 can be inserted in beneath the belt, wherewith the hook material 2 extending along the outer edges of the absorbent part coacts with the loop material 1 on the inside of the belt 15 to secure the diaper to the wearer. Alternatively, the loop material 1 may be provided on the absorbent part 14 and the hook material 2 on the belt 15. Another variant is to provide a fastener surface (either the male or female component) on the outside of the belt 15, and to provide the strip that carries either hook material or loop material on the inside of the absorbent body 14, i.e. on that side of said body that shall face towards the wearer in use. That part of the hook and loop fastener system provided on the belt 15 may either be a continuous strip, as shown in Fig. 10, or comprise several smaller pieces.

It will be understood that the uses described above have been given only by way of example and in no way limit the scope of the invention. The inventive fastener device can be used in all cases where a hook and loop fastener system that includes a male and female component is used.

**Claims**

1. Nonwoven fabric that includes a surface for fastening the male component of a hook and loop fastener system which comprises a needled fabric of functional fibres and binding fibres, where the functional fibres are comprised of thermoplastic polymer fibres, and where the nonwoven fabric is bonded by partially melting the binding fibres, **characterised in that** the needled fabric is loop-free and carded.

2. A nonwoven fabric according to Claim 1, **characterised in that** the functional fibres are comprised of one or more types of polyester fibres and/or polypropylene fibres.

3. A nonwoven fabric according to Claim 2, **characterised in that** the fabric includes two types of functional polyester fibres of mutually different thickness.

4. A nonwoven fabric according to any one of Claims 1-3, **characterised in that** the functional fibres include spiralled bi-component fibres or multi-component fibres of the side-by-side type.

5. A nonwoven fabric according to Claim 4, **characterised in that** the functional fibres include both crimped polyester fibres and spiralled fibres.

6. A nonwoven fabric according to any one of Claims 1-5, **characterised in that** the fabric contains 10-25% by weight binding fibres and 75-90% by weight functional fibres.

7. A nonwoven fabric according to any one of Claims 1-6, **characterised in that** 40-60% by weight of the functional fibres are spiralled fibres.

8. A nonwoven fabric according to any one of Claims 1-7, **characterised in that** the binding fibres comprise bi-component fibres that include a core and an outer casing, where the outer casing component has a lower melting point than the inner core component.

9. A method of producing a nonwoven fabric that has a fastener surface according to Claim 1, comprising the steps of carding a mixture of binding fibres and functional fibres comprised of thermoplastic polymer fibres to form a fibrous web; needling the fibrous web to obtain a dense material that has a loop-free, open structure suitable for the male component of a hook and loop fastener to fasten thereto; and heating the needled fibrous web so as to partially melt the binding fibres.

10. A method according to Claim 9, **characterised by** smooth calendering the needled and heated fibrous web so that one surface thereof will be smooth.

11. A method according to Claim 9 or 10, **characterised in that** the binding fibres are comprised of bi-component fibres that include a core and a casing, where said casing has a lower melting point than the core; and heating the needled fibrous web so that the casings of respective binding fibres will melt while the core remains solid.

12. An absorbent article (14), such as a diaper or incontinence guard, which includes a substantially liquid-impermeable backing sheet and a hook and loop fastener system that includes a female component and a male component attached to said backing sheet for mutual coaction such as to secure the article in position on a wearer, **characterised in that** the female component (1) of the system is comprised of nonwoven fabric according to any one of Claims 1-8.

13. An article according to Claim 12, **characterised in that** the article includes an absorbent body enclosed between a liquid-permeable inner sheet that lies proximal to the wearer in use and said substantially liquid-impermeable backing sheet that lies distal from the wearer in use, said backing sheet being delimited by two short sides (10, 11) and two long sides (12, 13), wherein two male-component tabs (2) belonging to said fastener system are fastened to said backing sheet on each long side (12, 13) thereof close to one short side (10), so as to form an extension of the short side with the male component facing in the same direction as the inner sheet; and **in that** the female component (1) is provided on the backing sheet at the other short side (11).

14. An article according to Claim 13, **characterised in that** one female component piece (1) is provided in each corner of said second short side (11).

15. An article according to Claim 13, **characterised in that** the female component (1) is comprised of a strip that extends essentially along the full length of the second short side (11).

16. An article according to Claim 12, **characterised in that** said article includes two parts, a belt (15) which is intended to be fastened around the waist of a wearer, and an absorbent part (14) which, in use, is fastened to the belt by means of mutually coacting male and female components (1, 2) of the hook and loop fastener system and which includes an absorbent body enclosed between a liquid-permeable inner sheet intended to lie proximal to the wearer in use and said substantially liquid-impermeable backing sheet intended to lie distal from the wearer in use and delimited by two short sides (10, 11) and two long sides (12, 13), wherein the mutually coacting components (1, 2) are disposed along the short sides (10, 11) of said absorbent body and on said belt (15) respectively.

17. An article according to Claim 16, **characterised in that** the female component (1) is disposed on the inner surface of the belt (15) and the male component (2) is disposed on the outer surface of the absorbent body (14).

18. An article according to Claim 16, **characterised in that** the male component (2) is disposed on the inner surface of the belt (15) and the female component (1) is disposed on the outer surface of the absorbent body (14).

19. An article according to Claim 16, **characterised in that** the male component (2) is disposed on the outer surface of the belt (15) and the female component (1) is disposed on the inner surface of the absorbent body (14).

20. An article according to Claim 16, **characterised in that** the female component (1) is disposed on the outer surface of the belt (15) and the male component (2) is disposed on the inner surface of the absorbent body (14).

21. The use of a nonwoven fabric according to any of claims 1-8 as the female component in a hook and loop fastener system in an absorbent article (14), such as a diaper or incontinence guard, which includes a substantially liquid-impermeable backing sheet and a hook and loop fastener system that includes a female component and a male component attached to said backing sheet for mutual coaction such as to secure the article in position on a wearer.

**Patentansprüche**

1. Vliesstoff, der eine Oberfläche zum Befestigen der männlichen Komponente eines Klettverschlusssystems, der ein genadeltes Tuch aus funktionalen Fasern und bindenden Fasern umfasst, wobei die funktionalen Fasern thermoplastische Polymerfasern enthalten und wobei der Vliesstoff durch teilweises Schmelzen der bindenden Fasern verbunden ist, **dadurch gekennzeichnet, dass** der genadelte Stoff schlaufenfrei und kardiert ist.

2. Vliesstoff nach Anspruch 1, **dadurch gekennzeichnet, dass** die funktionalen Fasern eine oder mehrere Arten von Polyesterfasern und/oder Polypropylenfasern enthalten.

3. Vliesstoff nach Anspruch 2, **dadurch gekennzeichnet, dass** der Stoff zwei Arten funktionaler Polyesterfasern gegenseitig unterschiedlichen Dickenmaßes beinhaltet.

4. Vliesstoff nach einem der Ansprüche 1 bis 3, **dadurch gekennzeichnet, dass** die funktionalen Fasern aufgewirbelte Bikomponentenfasern oder Multikomponentenfasern der Nebeneinander-Art (engl.: side-by-side type) beinhalten.

5. Vliesstoff nach Anspruch 4, **dadurch gekennzeichnet, dass** die funktionalen Fasern sowohl gekräuselte Polyesterfasern als auch aufgewirbelte Fasern beinhalten.

6. Vliesstoff nach einem der Ansprüche 1 bis 5, **dadurch gekennzeichnet, dass** der Stoff 10-25% Massenanteil bindende Fasern und 75-90% Massenanteil funktionale Fasern enthält.

7. Vliesstoff nach einem der Ansprüche 1 bis 6, **dadurch gekennzeichnet, dass** 40-60 % Massenanteil der funktionalen Fasern aufgewirbelte Fasern sind.

8. Vliesstoff nach einem der Ansprüche 1 bis 7, **dadurch gekennzeichnet, dass** die bindenden Fasern Bikomponentenfasern umfassen, die einen Kern und eine äußere Hülle aufweisen, wobei die äußere Hüllenkomponente einen niedrigeren Schmelzpunkt als die innere Kernkomponente aufweist.

9. Verfahren zum Herstellen eines Vliesstoffes nach Anspruch 1, der eine Befestigungsfläche aufweist, umfassend die Schritte Kardieren einer Mischung von bindenden Fasern und funktionalen Fasern, die thermoplastische Polymerfasern enthalten, um eine faserförmige Bahn zu bilden; Nadeln der faserförmigen Bahn, um ein dichtes Material zu erreichen, das eine schlaufenfreie, offene Struktur aufweist, die dazu geeignet ist, dass die männliche Komponente eines Klettverschlusses daran befestigt werden kann; und Erwärmen der genadelten faserförmigen Bahn, um so die bindenden Fasern teilweise zu schmelzen.

10. Verfahren nach Anspruch 9, **gekennzeichnet durch** weiches Kalendern der genadelten und erwärmten faserförmigen Bahn, so dass eine Oberfläche davon glatt wird.

11. Verfahren nach Anspruch 9 oder 10, **dadurch gekennzeichnet, dass** die bindenden Fasern Bikomponentenfasern enthalten, die einen Kern und eine Hülle aufweisen, wobei die Hülle einen niedrigern Schmelzpunkt aufweist als der Kern; und Erwärmen der genadelten faserförmigen Bahn, so dass die Hüllen der entsprechenden bindenden Fasern schmelzen, während der Kern fest bleibt.

12. Absorbierender Gegenstand (14), wie beispielsweise eine Windel oder ein Inkontinenzschutz, der eine im wesentlichen flüssigkeits-undurchlässige Deckschicht und ein Klettverschlusssystem, das eine weibliche Komponente und eine männliche Komponente umfasst, die zum gegenseitigen Zusammenwirken derart an der Deckschicht angebracht sind, um den Gegenstand in einer Position des Trägers festzulegen, aufweist, **dadurch gekennzeichnet, dass** die weibliche Komponente (1) des Systems ein Vliesstoff gemäß einem der Ansprüche 1 bis 8 enthält.

13. Gegenstand nach Anspruch 12, **dadurch gekennzeichnet, dass** der Gegenstand einen absorbierenden Körper aufweist, der zwischen einer flüssigkeitsdurchlässigen inneren Schicht, die bei Verwendung proximal des Trägers liegt und der im wesentlichen flüssigkeits-undurchlässigen Deckschicht, die bei der Verwendung distal des Trägers liegt, eingeschlossen ist, wobei die Deckschicht durch zwei kurze Seiten (10, 11) und zwei lange Seiten (12, 13) begrenzt ist und zwei männliche Komponentenstreifen (2), die zu dem Verschlusssystem gehören, an der Deckschicht an ihrer langen Seite (12, 13) nahe der einen kurzen Seite (10) befestigt sind, um so eine Verlängerung der kurzen Seite zu bilden, bei der die männliche Komponente in die gleiche Richtung weist wie die innere Schicht; und, dadurch, dass die weibliche Komponente (1) auf der Deckschicht an der anderen kurzen Seite (11) vorgesehen ist.

**14.** Gegenstand nach Anspruch 13, **dadurch gekennzeichnet, dass** ein weibliches Komponentenstück (1) in jeder Ecke der zweiten kurzen Seite (11) vorgesehen ist.

**15.** Gegenstand nach Anspruch 13, **dadurch gekennzeichnet, dass** die weibliche Komponente (1) einen Streifen umfasst, der sich im wesentlichen entlang der gesamten Länge der zweiten kurzen Seite (11) erstreckt.

**16.** Gegenstand nach Anspruch 12, **dadurch gekennzeichnet, dass** der Gegenstand zwei Teile umfasst, einen Gürtel (15), der dazu gedacht ist um die Taille eines Trägers befestigt zu werden und einen absorbierenden Teil (14), der bei Verwendung mittels gegenseitig zusammenwirkenden männlichen und weiblichen Komponenten (1, 2) des Klettverschlusssystems an dem Gürtel befestigt ist und welcher einen absorbierenden Körper aufweist, der zwischen einer flüssigkeitsdurchlässigen inneren Schicht, die dazu gedacht ist, bei Verwendung proximal des Trägers zu liegen und der im wesentlichen flüssigkeits-undurchlässigen Deckschicht, die dazu gedacht ist, bei Verwendung distal des Trägers zu liegen, eingeschlossen und durch zwei kurze Seiten (10, 11) und zwei lange Seiten (12, 13) begrenzt ist, wobei die gegenseitig zusammenwirkenden Komponenten (1, 2) entlang der kurzen Seiten (10, 11) des absorbierenden Körpers bzw. auf dem Gürtel (15) angeordnet sind.

**17.** Gegenstand nach Anspruch 16, **dadurch gekennzeichnet, dass** die weibliche Komponente (1) auf der inneren Fläche des Gürtels (15) und die männliche Komponente (2) auf der äußeren Fläche des absorbierenden Körpers (14) angeordnet sind.

**18.** Gegenstand nach Anspruch 16, **dadurch gekennzeichnet, dass** die männliche Komponente (2) auf der inneren Fläche des Gürtels (15) angeordnet ist und die weibliche Komponente (1) auf der äußeren Fläche des absorbierenden Körpers (14) angeordnet ist.

**19.** Gegenstand nach Anspruch 16, **dadurch gekennzeichnet, dass** die männliche Komponente (2) auf der äußeren Fläche des Gürtels (15) angeordnet ist und die weibliche Komponente (1) auf der inneren Fläche des absorbierenden Körpers (14) angeordnet ist.

**20.** Gegenstand nach Anspruch 16, **dadurch gekennzeichnet, dass** die weibliche Komponente (1) auf der äußeren Fläche des Gürtels (15) angeordnet ist und die männliche Komponente (2) auf der inneren Fläche des absorbierenden Körpers (14) angeordnet ist.

**21.** Verwendung eines Vliesstoffes nach einem der Ansprüche 1 bis 8 als weibliche Komponente eines Klettverschlusssystems bei einem absorbierenden Gegenstand (14), wie einer Windel oder einem Inkontinenzschutz, der eine im wesentlichen flüssigkeits-undurchlässige Deckschicht und ein Klettverschlusssystem, das eine weibliche Komponente und eine männliche Komponente aufweist, die zum gegenseitigen Zusammenwirken derart an der Deckschicht angebracht sind, dass sie den Artikel an einem Träger in Position festlegen, aufweist.

## Revendications

1. Tissu non tissé qui comporte une surface destinée à fixer le composant mâle d'un système de fermeture à crochets et à boucles, qui comprend un tissu aiguilleté de fibres fonctionnelles et de fibres de liaison, dans lequel les fibres fonctionnelles comportent en des fibres de polymère thermoplastique, et dans lequel le tissu non tissé est lié en faisant fondre partiellement les fibres de liaison, **caractérisé en ce que** le tissu aiguilleté est exempt de boucles et cardé.

2. Tissu non tissé selon la revendication 1, **caractérisé en ce que** les fibres fonctionnelles comprennent un ou plusieurs types de fibres de polyester et/ou de fibres de polypropylène.

**3.** Tissu non tissé selon la revendication 2, **caractérisé en ce que** le tissu comprend deux types de fibres de polyester fonctionnelles présentant des épaisseurs différentes les unes des autres.

**4.** Tissu non tissé selon l'une quelconque des revendications 1 à 3, **caractérisé en ce que** les fibres fonctionelles comprennent des fibres de forme hélicoïdale, à deux composants ou des fibres de forme hélicoïdale à plusieurs composants du type côte à côte.

**5.** Tissu non tissé selon la revendication 4, **caractérisé en ce que** les fibres fonctionnelles comprennent des fibres de polyester frisées et des fibres de forme hélicoïdale.

**6.** Tissu non tissé selon l'une quelconque des revendications 1 à 5, **caractérisé en ce que** le tissu renferme 10 à 25 % en poids de fibres de liaison et 75 à 90 % en poids de fibres fonctionnelles.

**7.** Tissu non tissé selon l'une quelconque des revendications 1 à 6, **caractérisé en ce que** 40 à 60 % en poids des fibres fonctionnelles sont des fibres de forme hélicoïdale.

**8.** Tissu non tissé selon l'une quelconque des revendications 1 à 7, **caractérisé en ce que** les fibres de liaison comprennent des fibres à deux composants qui comportent une âme et une enveloppe extérieure, le composant d'enveloppe extérieure ayant un point de fusion inférieur à celui du composant d'âme intérieure.

**9.** Procédé de fabrication d'un tissu non tissé qui comporte une surface de fermeture selon la revendication 1, comprenant les étapes consistant à carder un mélange de fibres de liaison et de fibres fonctionelles comportent en des fibres de polymère thermoplastique, pour former une bande constituée de fibres ; aiguilleter la bande constituée de fibres pour obtenir un matériau dense qui comporte une structure ouverte, exempte de boucles, adéquate pour que le composant mâle d'une fermeture à crochets et à boucles s'y attache ; et chauffer la bande constituée de fibres aiguilletée de manière à faire fondre partiellement les fibres de liaison.

**10.** Procédé selon la revendication 9, **caractérisé par** un calandrage modéré de la bande constituée de fibres aiguilletée et chauffée de façon telle que l'une de ses surfaces devienne douce.

**11.** Procédé selon la revendication 9 ou 10, **caractérisé en ce que** les fibres de liaison comprennent des fibres à deux composants qui comportent une âme et une enveloppe, ladite enveloppe ayant un point de fusion inférieur à celui de l'âme ; et **en ce que** le chauffage de la bande constituée de fibres aiguilletée est effectué de façon telle que les enveloppes des fibres de liaison respectives fondent tandis que l'âme reste solide.

**12.** Article absorbant (14), tel qu'une couche-culotte ou une protection contre l'incontinence, qui comprend une feuille arrière sensiblement imperméable aux liquides et un système de fermeture à crochets et à boucles qui comporte un composant femelle et un composant mâle fixé à ladite feuille arrière en vue de coopérer pour fixer l'article en place sur un utilisateur, **caractérisé en ce que** le composant femelle (1) du système est constitué par un tissu non tissé selon l'une quelconque des revendications 1 à 8.

**13.** Article selon la revendication 12, **caractérisé en ce que** l'article comprend un corps absorbant enfermé entre une feuille intérieure perméable aux liquides qui est située à proximité de l'utilisateur lors de l'utilisation et ladite feuille arrière sensiblement imperméable aux liquides qui est située à l'écart de l'utilisateur lors de l'utilisation, ladite feuille arrière étant délimitée par deux petits côtés (10, 11) et deux grands côtés (12, 13), deux pattes (2) de composant mâle appartenant audit système de fermeture étant fixées à ladite feuille arrière sur chacun de ses grands côtés (12, 13) tout près de l'un des petits côtés (10) de manière à réaliser un prolongement du petit côté, le composant mâle étant orienté dans la même direction que la feuille intérieure ; et **en ce que** le composant femelle (1) est prévu sur la feuille arrière sur l'autre petit côté (11).

**14.** Article selon la revendication 13, **caractérisé en ce qu'**une pièce (1) de composant femelle est prévue à chaque coin du deuxième petit côté (11).

**15.** Article selon la revendication 13, **caractérisé en ce que** le composant femelle (1) comprend une bande qui s'étend sensiblement sur toute la longueur du deuxième petit côté (11).

**16.** Article selon la revendication 12, **caractérisé en ce que** ledit article comprend deux parties, une ceinture (15) qui est destinée à être attachée autour de la taille d'un utilisateur et une partie absorbante (14) qui, lors de l'utilisation, est attachée à la ceinture au moyen de composants (1, 2) mâle et femelle, qui coopèrent l'un avec l'autre, du système de fermeture à crochets et à boucles, et qui comprend un corps absorbant enfermé entre une feuille intérieure perméable aux liquides destinée à se trouver très près de l'utilisateur lors de l'utilisation et ladite feuille arrière sensiblement imperméable aux liquides destinée à se trouver à l'écart de l'utilisateur lors de l'utilisation et délimité par deux petits côtés (10, 11) et deux grands côtés (12, 13), les composants (1, 2) coopérant l'un avec l'autre étant disposés le long des petits côtés (10, 11) respectivement dudit corps absorbant et de ladite ceinture (15).

17. Article selon la revendication 16, **caractérisé en ce que** le composant femelle (1) est disposé sur la surface intérieure de la ceinture (15) et le composant mâle (2) est disposé sur la surface extérieure du corps absorbant (14).

18. Article selon la revendication 16, **caractérisé en ce que** le composant mâle (2) est disposé sur la surface intérieure de la ceinture (15) et **en ce que** le composant femelle (1) est disposé sur la surface extérieure du corps absorbant (14).

19. Article selon la revendication 16, **caractérisé en ce que** le composant mâle (2) est disposé sur la surface extérieure de la ceinture (15) et **en ce que** le composant femelle (1) est disposé sur la surface intérieure du corps absorbant (14).

20. Article selon la revendication 16, **caractérisé en ce que** le composant femelle (1) est disposé sur la surface extérieure de la ceinture (15) et **en ce que** le composant mâle (2) est disposé sur la surface intérieure du corps absorbant (14).

21. Utilisation d'un tissu non tissé selon l'une quelconque des revendications 1 à 8 en tant que composant femelle dans un système à crochets et à boucles dans un article absorbant (14) tel qu'une couche-culotte ou une protection contre l'incontinence, qui comprend une feuille arrière sensiblement imperméable aux liquides et un système de fermeture à crochets et à boucles qui comprend un composant femelle et un composant mâle fixé à ladite feuille arrière en vue de coopérer l'un avec l'autre de manière à fixer l'article en place sur un utilisateur.

FIG.1

FIG.2A

FIG.2B

FIG.3A

FIG.4

FIG.3B

FIG.5

FIG.6

FIG.7

FIG. 8

FIG. 9

FIG.10